# EUROPEAN PATENT APPLICATION

(11) **EP 2 814 081 A2**
(43) Date of publication of application: **17.12.2014**
(21) Application number: 13177247.7
(22) Date of filing: 19.07.2013
(51) Int. Cl.: H01M 2/10, A61M 15/06, H01M 2/20

(54) **Battery assembly and electronic cigarette**

(30) Priority: 14.06.2013 CN 201310233243
(71) Applicant: Shenzhen First Union Technology Co., Ltd., Shenzhen Guangdong (CN)
(72) Inventor: Li, Yonghai, Shenzhen Guangdong (CN); Xu, Zhongli, Shenzhen Guangdong (CN)
(74) Representative: Zenz

(57) **Abstract**

A battery assembly of an electronic cigarette includes a battery sleeve, a battery received in the battery sleeve, an electrode ring arranged at an end of the battery, an elastic member and an insulated ring received in the battery sleeve. The insulated ring defines a through hole exposed along an axial direction of the battery sleeve, and the electrode ring is resiliently arranged in the through hole by the elastic member and extending through the through hole. A battery assembly includes an atomizing device and the battery assembly.

## Description

### 1. Technical Field

The present invention relates to electronic cigarettes, and particularly to a battery assembly and an electronic cigarette using same.

### 2. Description of Related Art

Electronic cigarettes are similar to conventional cigarettes in appearance and taste, but less harmful to human's health, such that electronic cigarettes are widely used for helping people to quit smoke. A typical electronic cigarette includes an atomizing device and a battery assembly. When users smoke at an end of the atomizing device, the battery assembly powers the atomizing device, then the users can have the smoking feelings.

In current battery assemblies, electrode rings used to be electrically connected to the atomizing device are usually fixed at an end of the battery assemblies, such that in some situations, the electrode rings may be not well connected to the atomizing devices due to position differences in different battery assemblies.

What is needed, therefore, is a battery assembly with a resilient electrode ring and an electronic cigarette using same, which can overcome the above shortcomings.

### BRIEF DESCRIPTION OF THE DRAWINGS

Many aspects of the present battery assembly and electronic cigarette can be better understood with reference to the following drawings. The components in the drawings are not necessarily drawn to scale, the emphasis instead being placed upon clearly illustrating the principles of the present battery assembly and electronic cigarette. Moreover, in the drawings, like reference numerals designate corresponding parts throughout the several views.

FIG. 1 is an isometric schematic view of an electronic cigarette in accordance with a first embodiment.

FIG. 2 is a cross sectional view of a battery assembly of the electronic cigarette of FIG. 1 taken along a central line of the battery assembly.

FIG. 3 is similar to FIG. 2, but taken along another central line of the battery assembly.

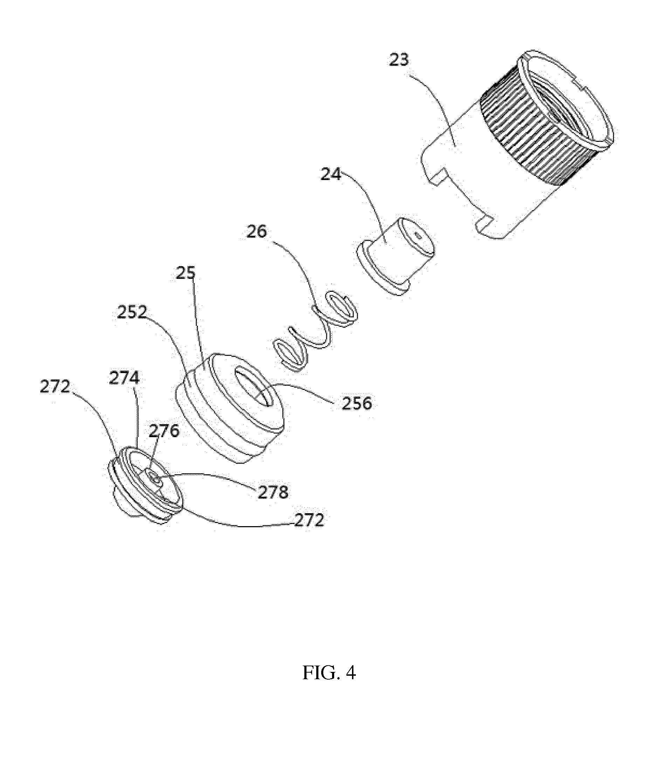

FIG. 4 is an isometric schematic view of a screw sleeve, an electrode ring, an insulated ring, an elastic member, and a fixing base shown in FIG. 2.

FIG. 5 is a cross sectional view of a battery assembly of an electronic cigarette in accordance with a second embodiment.

FIG. 6 is a cross sectional view of a battery assembly of an electronic cigarette in accordance with a third embodiment.

FIG. 7 is similar to FIG. 6, but taken along another central line of the battery assembly.

FIG. 8 is a cross sectional view of a battery assembly of an electronic cigarette in accordance with a fourth embodiment.

### DETAILED DESCRIPTION

Embodiments of the present battery assembly and electronic cigarette will now be described in detail below and with references to the drawings.

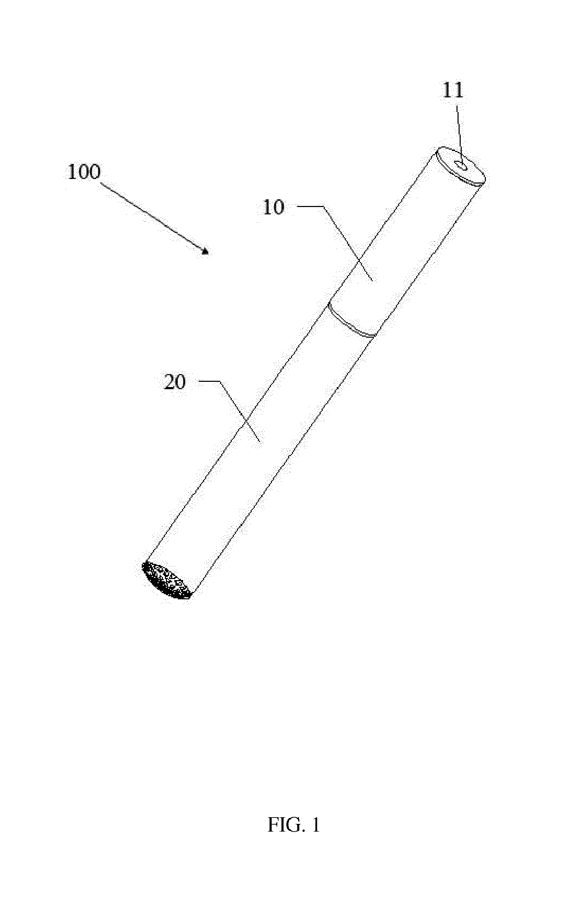

Referring to FIG.1, an electronic cigarette 100 includes an atomizer 10 and a battery assembly 20. One end of the atomizer 10 has a suction nozzle 11, and the other end of the atomizer 10 is connected to the battery assembly 20.

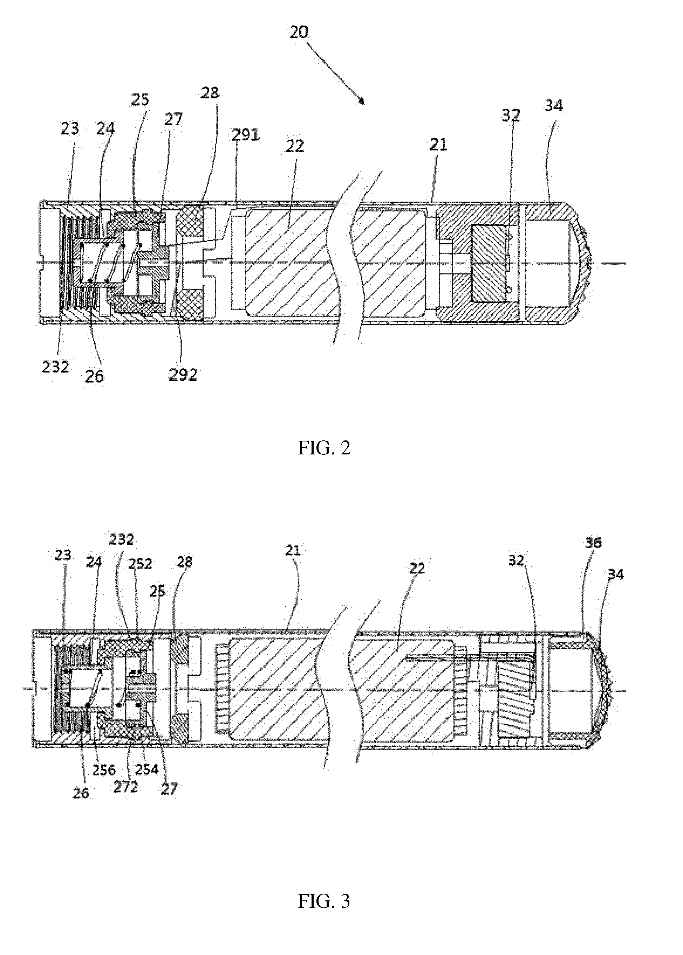

Referring to FIGS. 2 and 3, the battery assembly 20 mainly includes a battery sleeve 21, a battery 22 received in the battery sleeve 21, a screw sleeve 23 located at an end of the battery 22, an electrode ring 24, an insulated ring 25, an elastic member 26, a fixing base 27 and a ring-shaped silicone rubber spacer 28.

The battery 22 is a storage battery, and can be charged in need. The battery sleeve 21 and the screw sleeve 23 are all made of metallic materials, and the screw sleeve 23 is fixed in the battery sleeve 21. In the present embodiment, the screw sleeve 23 has inner screws 232, and is connected to the atomizer 10 by the inner screws 232.

The insulated ring 25 is fixed in the screw sleeve 23, and is made of an insulated material, such as a plastic material. The insulated ring 25 has a through hole 256 defined through the insulated ring 25. In the present embodiment, the elastic member 26 is an elastic member.

The electrode ring 24, elastic member 26 and fixing base 27 are received in the through hole 256. The electrode ring 24 extends through the through hole 256 towards the atomizer 10 and is fixed at the fixing base 27 via the elastic member 26. The through hole 256 has a certain depth, and the electrode ring 24 is spaced a certain distance from the fixing base 27, thereby the electrode ring 24 can be moved upwards and downwards due to the elastic member 26. In the present embodiment, the insulated ring 25 has a ring-shaped protrusion 252 at an outer wall thereof, and the screw sleeve 23 has a ring-shaped recess 232 at an inner wall thereof, thereby fixing the insulated ring 25 in the screw sleeve 23; in addition , the fixing base 27 has a ring-shaped recess 272 at an outer wall thereof, and the insulated ring 25 has a ring-shaped protrusion 254 at an inner wall thereof, thereby fixing the fixing base 27 in the insulated ring 25.

The fixing base 27 includes a bottom plate 272, a circular side wall 274 extending upwards from the bottom plate 272, and an annular projection 276 projected upwards and downwards from a central position of the bottom plate 272. The annular projection 276 has an air through hole 278 defined in a center thereof. The bottom plate 272 and the circular side wall 274 cooperatively form a container configured for receiving oil leaked from the atomizing device 10, thereby avoiding pollution to the battery 22.

The fixing base 27 is made of a metallic material. The electrode ring 24, the elastic member 26 and the fixing base 27 are electrically contacted one by one. The fixing base 27 is electrically connected to one pole, preferably the negative pole of the battery 22 via a wire 291, and the wire 291 can be welded to a bottom of the fixing base 27. Another pole, preferably the positive pole of the battery 22 can be electrically connected to the screw sleeve 23 via a wire 292.

The silicone rubber spacer 28 is positioned at a distal end of the screw sleeve 23, and is located between the screw sleeve 23 and the battery 22. The silicone rubber spacer 28 is configured for avoiding the screw sleeve 23 and other elements pressing the battery 22.

The battery assembly 20 further has a light emitting element 32 and a cover 34 with at least one air through hole 36. Light can be transmitted through the cover 34, such that when a user sucks the nozzle 11 of the atomizing device 10, air flow makes the battery assembly 20 power the atomizing device 10, and the light emitting element 32 emit light at the same time. Light then can transmit through the cover 34, such that the electronic cigarette 100 can have a conventional cigarette burning feeling.

Concluded from the above description, the electrode ring 24 is fixed in the through hole 256 via the elastic member 26. Due to the elasticity of the elastic member 26 and a depth of the through hole 256, a connection depth between the electrode ring 24 and the atomizing device 10 can be adjusted, thereby the connection of battery assembly 20 and the atomizing device 10 is easy, and the battery assembly 20 can be applicable for various atomizing devices 10. The battery assembly 20 therefore may be assembled automatically by machine. The fixing base 27 can avoid pollution of the oil from the atomizing device 10 to the battery 22.

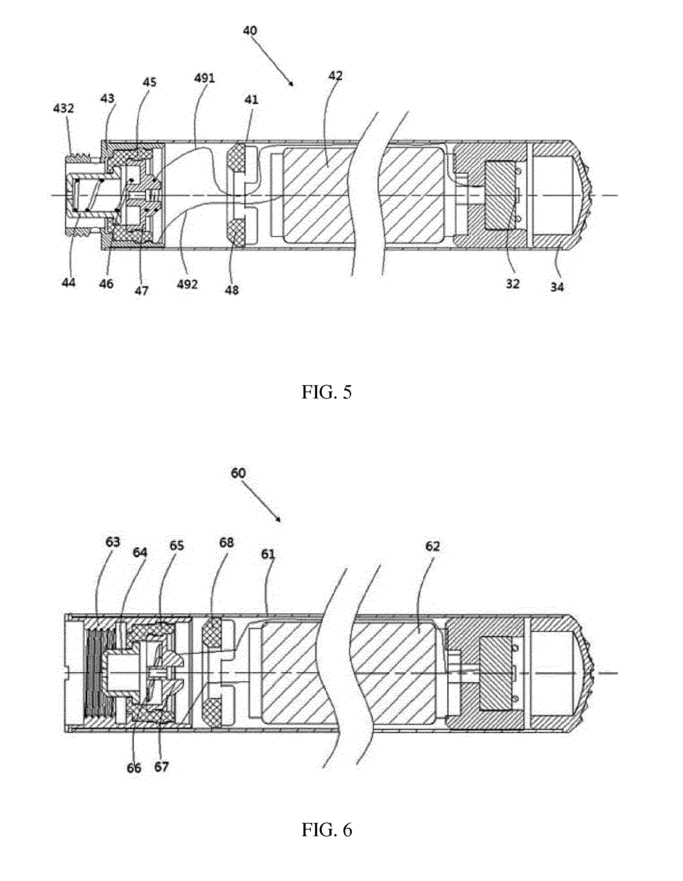

Referring to FIG. 5, a battery assembly 40 of an electronic cigarette in accordance with a second embodiment is provided. The battery assembly 40 includes a battery sleeve 41, a battery 42, a screw sleeve 43, an electrode ring 44, an insulated ring 45, an elastic member 46, a fixing base 47 and a ring-shaped silicone rubber spacer 48. One pole of the battery 42 is electrically connected to the fixing base 47 via a wire 491 welded to a bottom of the fixing base 47, and another pole of the battery 42 is electrically connected to the screw sleeve 43 via a wire 492. The battery assembly 40 also has a light emitting element 32 and a cover 34.

The difference between the battery assembly 40 of the second embodiment and the battery assembly 20 of the first embodiment is that the screw sleeve 43 has outer screws 432, correspondingly a connection end of the atomizing device has inner screws, such that the battery assembly is threadedly connected to the atomizing device.

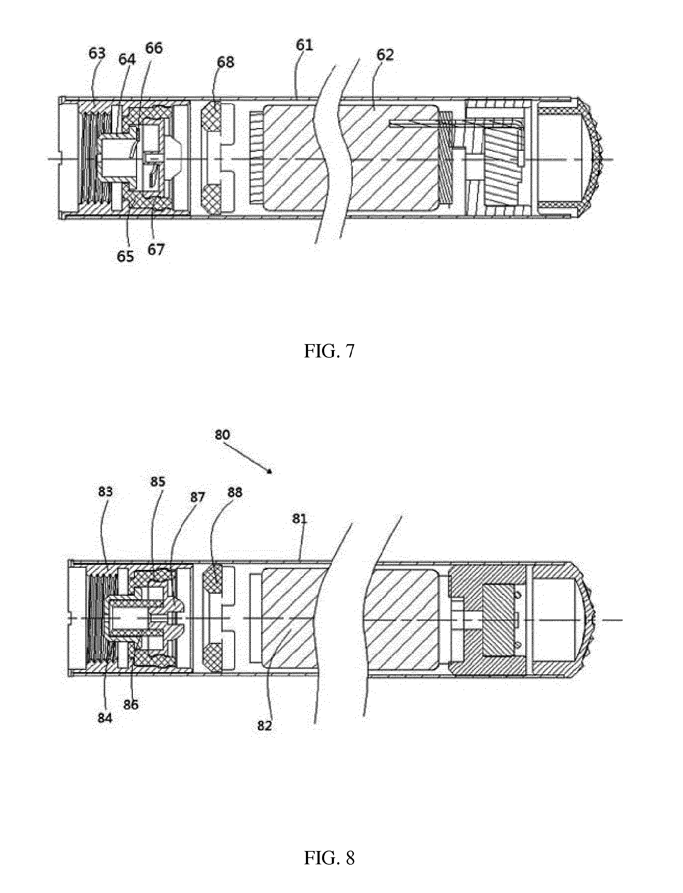

Referring to FIG. 6 and FIG. 7, a battery assembly 60 of an electronic cigarette in accordance with a third embodiment is provided. The difference between the battery assembly 60 of the third embodiment and the battery assembly 20 of the first embodiment is that an elastic member 66 of the battery assembly 60 is a resilient sheet. The resilient sheet can be electrically conductive. In other embodiments, the resilient sheet is insulated, and wires can be applied to the electrode ring 64 for electrical connection. A battery sleeve 61, a battery 62, a screw sleeve 63, an electrode ring 64, an insulated ring 65, a fixing base 67 and a silicone rubber spacer 68 of the battery assembly 60 are the same as the elements in the battery assembly 20 of the first embodiment.

Referring to FIG. 8, a battery assembly 80 of an electronic cigarette of a fourth embodiment is provided. The difference between the battery assembly 80 of the fourth embodiment and the battery assembly 20 of the first embodiment is that an elastic element 86 of the battery assembly 80 is a silicon rubber ring. The silicon rubber ring is preferably electrically conductive. A battery sleeve 81, a battery 82, a screw sleeve 83, an electrode ring 84, an insulated ring 85, a fixing base 87 and a silicone rubber spacer 88 of the battery assembly 80 are the same as the elements in the battery assembly 20 of the first embodiment.

In other embodiments, the elastic element may be a fibre ring, hydraulic device or other rubber rings. The elastic element can be electrically conductive or insulated, and wires can be applied to electrode ring when the elastic element is insualted.

It is understood that the above-described embodiments are intended to illustrate rather than limit the disclosure. Variations may be made to the embodiments and methods without departing from the spirit of the disclosure. Accordingly, it is appropriate that the appended claims be construed broadly and in a manner consistent with the scope of the disclosure.

## Claims

1. A battery assembly of an electronic cigarette, comprising:
a battery sleeve;
a battery received in the battery sleeve;
an electrode ring arranged at an end of the battery;
an elastic member; and
an insulated ring received in the battery sleeve, the insulated ring comprising a through hole exposed along an axial direction of the battery sleeve, the electrode ring resiliently arranged in the through hole by the elastic member and extending through the through hole.

2. The battery assembly of claim 1, wherein the elastic member is selected from a group consisting of spring, resilient sheet, a silicone rubber spacer, a fibre ring and a hydraulic device.

3. The battery assembly of claim 2, wherein the elastic member is electrically conductive.

4. The battery assembly of claim 1, further comprising a screw sleeve fixed in the battery sleeve at an end of the battery, the insulated ring being fixed in the screw sleeve.

5. The battery assembly of claim 4, further comprising an electrically conductive fixing base received in the through hole, the elastic member being fixed on the fixing base, the fixing base and the screw sleeve being respectively electrically connected to two poles of the battery.

6. The battery assembly of claim 5, wherein the fixing base comprises a bottom plate, a circular side wall extending from the bottom plate, and an annular projection projected from a central area of the bottom plate, the annular projection having an air through hole defined therein.

7. An electronic cigarette, comprising:
an atomizing device; and
a battery assembly comprising:
a battery sleeve;
a battery received in the battery sleeve;
an electrode ring arranged at an end of the battery;
an elastic member; and
an insulated ring received in the battery sleeve, the insulated ring comprising a through hole exposed along an axial direction of the battery sleeve, the electrode ring resiliently arranged in the through hole by the elastic member and extending through the through hole to be connected to the atomizing device.

8. The electronic cigarette of claim 7, wherein the elastic member is selected from a group consisting of spring, resilient sheet, a silicone rubber spacer, a fibre ring and a hydraulic device.

9. The electronic cigarette of claim 7, further comprising a screw sleeve fixed in the battery sleeve at an end of the battery, and an electrically conductive fixing base, the insulated ring being fixed in the screw sleeve, the fixing base being fixed in the through hole of the insulated ring, the elastic member resiliently fixed on the fixing base, the fixing base and the screw sleeve being respectively electrically connected to two poles of the battery via wires.

10. The electronic cigarette of claim 9, wherein the fixing base comprises a bottom plate, a circular side wall extending from the bottom plate, and an annular projection projected from a central area of the bottom plate, the annular projection having an air through hole defined therein.
